# EUROPÄISCHE PATENTSCHRIFT

(11) **EP 3 287 014 B1**
(45) Veröffentlichungstag und Bekanntmachung des Hinweises auf die Patenterteilung: **26.02.2020**
(21) Anmeldenummer: 16185185.2
(22) Anmeldetag: 22.08.2016
(51) Int. Cl.: A23L 33/11, A61K 31/575, C11B 13/00, C11C 3/12, A23L 5/20

(54) **VERFAHREN ZUR HERSTELLUNG EINER PHYTOSTEROL-PHYTOSTANOLZUSAMMENSETZUNG**
PROCESS FOR PRODUCING A PHYTOSTEROL-PHYSTOSTANOL COMPOSITION
PROCÉDÉ DE PRÉPARATION D'UNE COMPOSITION PHYTOSTEROL-PHYTOSTANOL

(43) Veröffentlichungstag der Anmeldung: 28.02.2018
(73) Patentinhaber: Verbio Vereinigte Bioenergie AG, 04109 Leipzig (DE)
(72) Erfinder: Lemp, Joachim, 04509 Delitzsch (DE); Pöhls, Emanuel, 04357 Leipzig (DE)
(74) Vertreter: Müller & Schubert

(56) Entgegenhaltungen:
- DE-T2- 69 632 481
- JP-A- 2001 335 795

## Beschreibung

Die vorliegende Erfindung betrifft ein Verfahren zur Herstellung einer Phytosterol-Phytostanolzusammensetzung, die mittels des Verfahrens hergestellte Zusammensetzung sowie deren Verwendung.

Phytosterole und Phytostanole werden als Nahrungsmittel-Zusatzstoffe verwendet, da ihr Verzehr die Aufnahme von Cholesterin und in Folge den Serum-Cholesterinspiegel (insbesondere LDL-Cholesterin) des Blutes signifikant zu senken vermag. Hauptquellen für Phytosterole und Phytostanole sind Pflanzenöl-Dämpferdestillate (aus der Speiseölgewinnung) und Tallöl (aus der Zellstoffproduktion).

Üblicherweise werden als Zusatzstoffe für Nahrungsmittel Zubereitungen auf Basis von hochreinen Phytosterol-Phytostanolkonzentraten eingesetzt. Diese Konzentrate werden durch Isolierung der Phytosterole und Phytostanole aus Pflanzenöl bzw. Tallöl unter Einsatz verschiedener Verfahrensschritte, wie z. B. Destillation, Extraktion, Kristallisation und Wäsche gewonnen. Diese Konzentrate enthalten eine Vielzahl verschiedener Phytosterole und Phytostanole. Je nachdem, aus welcher Pflanze bzw. welchem Pflanzenöl die Phytosterol-Phytostanolkonzentrate gewonnen werden, besitzt das jeweilige Konzentrat eine charakteristische Phytosterol-Phytostanolzusammensetzung, auch als Phytosterol-Phytostanolspektrum bezeichnet.

Im Zusammenhang der vorliegenden Erfindung umfasst der Begriff Phytosterol und Phytostanol neben den freien Sterolen und Stanolen auch weitere Sterolderivate und Stanolderivate, welche an der in der Position 3 des jeweiligen Grundgerüsts befindlichen Hydroxylgruppe substituiert sind. Unter Substitution der Hydroxylgruppe wird in diesem Zusammenhang beispielsweise die Umsetzung der Hydroxylgruppe mit einer Carboxylgruppe unter Ausbildung eines Esters verstanden. Unter Phytostanol und Phytosterol werden deshalb im Nachfolgenden auch Sterolester und Stanolester verstanden, die beispielsweise durch Umsetzung der Hydroxylgruppe mit kurzkettigen aliphatischen Carbonsäuren, wie zum Beispiel Ameisensäure, Essigsäure, Buttersäure und Propionsäure, oder pflanzenölstämmigen Fettsäuren erhalten werden.

Weiterhin wird unter Substitution in diesem Zusammenhang die Umsetzung der Hydroxylgruppe in 3-Position unter Ausbildung einer Ethergruppe verstanden. Hierbei ist eine Umsetzung mit einem Zucker unter Ausbildung eines Sterol- oder Stanolglykosids bevorzugt. In den Sterol- und Stanolglykosiden ist das Phytosterol oder Phytostanol als Aglykon durch ein Sauerstoffatom in einer als glykosidische Bindung bezeichneten Etherbindung an ein Halbacetal-Kohlenstoffatom des betreffenden Zuckers gebunden. Die Zucker sind beispielsweise ausgewählt aus Allose, Altrose, Glucose, Mannose, Gulose, Idose, Galactose, Talose und Fructose, sind jedoch nicht auf diese beschränkt.
Unter Phytostanol und Phytosterol werden deshalb im Nachfolgenden auch Sterolether und Stanolether verstanden.

Im Zusammenhang der Erfindung werden für die Phytosterole und Phytostanole auch Trivialnamen verwendet. Diese Trivialnamen werden nachfolgend aufgeführt und die chemischen Namen angegeben:
β-Sitosterol für (3β)-Stigmast-5-en-3-ol,
Stigmastanol für (3β,5α)-Stigmastan-3-ol,
Campesterol für (3β)-Ergost-5-en-3-ol,
Campestanol für (3β,5α)-Ergostan-3-ol,
Stigmasterol für (3β)-Stigmasta-5,22-dien-3-ol,
Brassicasterol für (3β)-Ergosta-5,22-dien-3-ol,
Ergosterol für (3β)-Ergosta-5,7,22-trien-3-ol,
wobei in der Literatur synonym für Stigmastanol auch der Trivialname Sitostanol und synonym für Campestanol auch der Trivialname Ergostanol verwendet wird.

Die genannten Phytosterole und Phytostanole weisen Chiralitätszentren auf und kommen in der Natur, also auch in Pflanzenöl, als Epimere vor. Bei den im Stand der Technik verwendeten und anerkannten Analyseverfahren werden die Epimere üblicherweise nicht voneinander getrennt. Wenn im Zusammenhang der vorliegenden Erfindung quantitative Angaben zu Phytosterolen und Phytostanolen gemacht werden, so wird, wie im Stand der Technik üblich, gegebenenfalls nicht zwischen deren Epimeren unterschieden.

Die nachfolgend verwendeten Prozentangaben der Phytosterol-Phytostanolzusammensetzungen sind, sofern nichts anderes angegeben ist, Gewichts-%, bezogen auf die Gesamtmasse an Phytosterolen und Phytostanolen in der Zusammensetzung. Hierbei werden die Anteile der Phytosterole und Phytostanole in ihrer freien Form bestimmt. So bezieht sich beispielsweise die Angabe "40 Gewichts-% Sitosterol" bei einem Phytosterol-Phytostanolkonzentrat auf die freien Sterole und Stanole, die demzufolge zu 40 Gewichts-% Sitosterol umfassen.

In den offenbarten Zusammensetzungen können die Phytosterole und Phytostanole derivatisiert oder in freier, also nicht-derivatisierter Form vorliegen. Derivatisiert bedeutet in diesem Zusammenhang, dass am Phytosterol- beziehungsweise am Phytostanolgerüst die in der 3-Position befindliche Hydroxylgruppe substituiert wurde. Die Substitution der Hydroxylgruppe erfolgt bevorzugt durch Umsetzung mit einer Carbonsäure, gegebenenfalls einem aktivierten Carbonsäurederivat, unter Ausbildung von Phytosterol- beziehungsweise Phytostanolestern. Weiterhin umfasst die Derivatisierung der Hydroxylgruppe in 3-Position auch die Bildung von Ethern, beispielsweise durch Reaktion des Phytostanols oder Phytosterols mit einem Zucker unter Ausbildung von Sterol- oder Stanolglykosiden.

Für die quantitative Bestimmung der Phytosterole und Phytostanole der Phytosterol-Phytostanolzusammensetzungen, die mittels des erfindungsgemäßen Verfahrens hergestellt werden bzw. in diesem als Edukt eingesetzt werden, wird das in der Sterol- und Stanol-Analytik gebräuchliche GC-FID-Verfahren verwendet, welches beispielsweise als *European Official Method of Analysis* in der *Commission Regulation EEC* 2568/1991 im Anhang V (5.1) beschrieben ist. Ein weiteres, allgemein anerkanntes Analyseverfahren für Sterole und Stanole ist aufgeführt in der Publikation der JECFA (Joint FAO/WHO Expert Committee on Food Additives) 2008, publiziert in FAO JECFA Monographs 5 (2008*).*

Beide Routine-GC-Bestimmungsverfahren, die anerkannter Stand der Technik in diesem Bereich sind, ermöglichen nicht die Unterscheidung zwischen gegebenenfalls vorliegenden Epimeren der Phytosterole bzw. Phytostanole. Deshalb versteht sich in den folgenden Ausführungen der jeweils benutzte Trivialname für die Phytosterole und Phytostanole als Gesamtheit der Epimere, welche mit der beschriebenen Analysenmethode die gleiche Retentionszeit wie das bezeichnete Epimer haben.

Dies trifft beispielsweise auf die folgenden Epimere der Phytosterole und Phytostanole zu: Bei Brassicasterol sind dies die Epimere (22E)-(24S)-Ergost-5,22-dien-3β-ol und (22E)-(24R)-Ergost-5,22-dien-3β-ol, bei Campesterol die Epimere (24R)-Ergost-5-en-3β-ol und (24S)-Ergost-5-en-3β-ol, bei Campestanol die Epimere (24R)-Ergostan-3β-ol und (24S)-Ergostan-3β-ol, bei Stigmasterol die Epimere (22E)-(24S)-Stigmast-5,22-dien-3β-ol und (22E)-(24R)-Stigmast-5,22-dien-3β**-**ol, bei Sitosterol die Epimere (22E)-(24R)-Stigmast-5-en-3β-ol und (22E)-(24S)-Stigmast-5-en-3β-ol und bei Stigmastanol die Epimere (24R)-Stigmastan-3β-ol und (24S)-Stigmastan-3β-ol.

Die bedeutendsten Quellen von Phytosterolen und Phytostanolen sind heutzutage Sojaöl sowie Tallöl. Phytosterol-Phytostanolkonzentrate aus diesen Quellen zeichnen sich durch hohe Gehalte am physiologisch bedeutsamen β-Sitosterol (bis zu 80 Gewichts-%) und äußerst geringe Gehalte an Brassicasterol (< 1 Gewichts-%) aus. Üblich für sojaölstämmige Phytosterol-Phytostanolkonzentrate sind < 25 Gewichts-% Campesterol und 14 bis 20 Gewichts-% Stigmasterol. Charakteristisch für tallölstämmige Phytosterol-Phytostanolkonzentrate ist weiterhin, dass sie < 1 Gewichts-% Stigmasterol, 10 bis 15 Gewichts-% Stigmastanol, 8 bis 11 Gewichts-% Campesterol und 1 bis 4 Gewichts-% Campestanol umfassen.

Als Zusatzstoffe für Nahrungsmittel unterliegen auch Phytosterol-Phytostanolkonzentrate Zulassungsverfahren. Basis für die Zulassung in der Europäischen Union ist die Verordnung (EG) Nr. 258/97 über neuartige Lebensmittel und Lebensmittelzutaten, ergänzt durch die entsprechenden Kommentare und Stellungnahmen des Scientific Committee on Food (SCF 2000, 2002 und 2003).

Auf Grundlage wissenschaftlicher Studien erachtet das Scientific Committee on Food (SCF) der Europäischen Kommission in seiner Stellungnahme vom 5. März 2003 zur Zulassung von Phytosterol-angereicherten Lebensmitteln ein Phytosterol-Phytostanolkonzentrat folgender Zusammensetzung als generell akzeptabel: ≤ 80 Gewichts-% β-Sitosterol, ≤ 15 Gewichts-% Stigmastanol, ≤ 40 Gewichts-% Campesterol, ≤ 5 Gewichts-% Campestanol, ≤ 30 Gewichts-% Stigmasterol, ≤ 3 Gewichts-% Brassicasterol und ≤ 3 Gewichts-% sonstige Phytosterole. Die Limitierung des Brassicasterolgehaltes ist darin begründet, dass in den herangezogenen toxikologischen Studien ausschließlich Phytosterol-Phytostanolmischungen untersucht wurden, die ≤ 3 Gewichts-% Brassicasterol in ihrem Spektrum aufwiesen.

Aufgrund dieser Vorgaben des SCF sind gegenwärtig rapsölstämmige Phytosterol-Phytostanolzusammensetzungen als Zusatzstoffe für Nahrungsmittel von untergeordneter Bedeutung, insbesondere aufgrund ihres hohen Gehaltes an Brassicasterol. So besitzt ein typisches hochreines rapsölstämmiges Phytosterol-Phytostanolkonzentrat beispielsweise folgende Zusammensetzung: 40 bis 60 Gewichts-% β-Sitosterol, 5 bis 13 Gewichts-% Brassicasterol, 25 bis 40 Gewichts-% Campesterol, ≤ 1 Gewichts-% Stigmasterol, ≤ 1 Gewichts-% Stigmastanol und ≤ 1 Gewichts-% Campestanol.

Rapsölstämmige Phytosterol-Phytostanolkonzentrate können daher in Nahrungsmitteln nur in begrenzten Mengen im Blend, also in Mischung mit z. B. tallöl- beziehungsweise sojaölstämmigen Phytosterol-Phytostanolkonzentraten verwendet werden.

Die Tatsache, dass rapsölstämmige Phytosterol-Phytostanolkonzentrate nur in geringem Umfang in Nahrungsmitteln verwendet werden können, ist nachteilig, weil rapsölstämmige Phytosterol-Phytostanolkonzentrate in größeren Mengen zur Verfügung gestellt werden können. So wird Rapsöl in großem Umfang als Nahrungsmittel und Ausgangsstoff für die Herstellung von Biodiesel (FAME) verwendet.

Bei der Raffination von Rapsöl fallen Dämpferdestillate als Nebenprodukte der Desodorierung/physikalischen Entsäuerung an. Diese Dämpferdestillate umfassen neben freien Fettsäuren und deren Mono-, Di- und Triglyceriden auch Phytosterole und Phytostanole. Dämpferdestillate aus der Desodorierung von Rapsöl dienen deshalb als eine Quelle zur Gewinnung von rapsölstämmigen Phytosterolen und Phytostanolen.
Es sind verschiedene Verfahren zur Isolierung von Phytosterolen und Phytostanolen aus Dämpferdestillaten bekannt und ein Verfahren wird beispielsweise im Patent US 5 424 457 beschrieben.

Bei der Herstellung von Biodiesel aus Rapsöl werden die im Rapsöl enthaltenen Glycerinester von Fettsäuren in die entsprechenden Methylester (RME) überführt. Bei einer anschließenden destillativen Aufarbeitung des Reaktionsprodukts dieser Umesterung des Rapsöls verbleibt ein Destillationsrückstand, in welchem die rapsölstämmigen Phytosterole und Phytostanole enthalten sind. Aus diesem Destillationsrückstand können die Phytosterole und Phytostanole als Konzentrat isoliert werden. Ein Verfahren zur Gewinnung von Phytosterolen aus Rückständen einer Destillation von Estern pflanzlicher Fettsäuren und/oder Ölen, vorzugsweise aus Destillationsrückständen aus einer Umesterung von pflanzlichen Ölen, wird beispielsweise in der europäischen Patentanmeldung EP 2 635 592 offenbart.

Um die auf dem Markt zur Verfügung stehenden rapsölstämmigen Phytosterol-Phytostanolkonzentrate nicht ausschließlich im Blend mit Phytosterolen und Phytostanolen aus anderen pflanzlichen Quellen als Zusatzstoff in Lebensmitteln nutzbar zu machen, besteht die Notwendigkeit, das Phytosterol-Phytostanolspektrum der Konzentrate an die Vorgaben des SCF anzupassen.

Da die Bereitstellung einer derart angepassten rapsölstämmigen Phytosterol-Phytostanolzusammensetzung jedoch möglichst kostengünstig und deshalb mit geringem technischen Aufwand erfolgen sollte, um wirtschaftlich vertretbar zu sein, sind im Stand der Technik bekannte Verfahren zur Abtrennung und Auftrennung der Phytosterol-Phytostanolkomponenten nachteilig.

So ist eine destillative Trennung eines Phytosterol-Pytostanolkonzentrats mittels fraktionierender Destillation nachteilig, da einerseits die Siedepunkte der Sterole zu eng beieinander liegen für eine ausreichende Trennung, andererseits Sterole äußerst temperaturempfindlich sind. Wie bedingt destillative Verfahren Sterolmischungen lediglich zu trennen in der Lage sind, wird in der Offenbarung des Patents US 3 879 431 deutlich.

Stand der Technik zur Veränderung der Sterol-Stanol-Spektren in Phytosterol-Pytostanolkonzentraten im industriellen Maßstab sind Verfahren zur fraktionierenden Lösemittelkristallisation. Ein historisches Verfahren stellt das von Upjohn dar, beschrieben in Industrial & Engineering Chemistry, Vol. 53, No. 12, Dezember 1961, Seiten 949-962. Dieser vielstufige Fraktionierprozess ist verfahrenstechnisch sehr aufwändig, die Erzeugung einer Stigmasterol-abgereicherten Phase ist mit hohen Verlusten verbunden, die Produktausbeute ist entsprechend gering. Da das Verfahren auf den unterschiedlichen Löslichkeiten/Schmelzpunkten von β-Sitosterol und Stigmasterol fußt und auf sojaölstämmige Sterolkonzentrate optimiert wurde, ist eine Anwendung auf rapsölstämmige Konzentrate nicht ohne weiteres möglich.

Ein ebenfalls auf Lösemittelkristallisation beruhendes Verfahren zur Abtrennung von Stigmasterol von Campesterol und β-Sitosterol wird beschrieben in Separation Science and Technology, Vol. 41, No. 13, September 2006, Seiten 3027-3038. Bei diesem Verfahren ist nachteilig, dass eine Stigmasterol angereicherte Phase als Koppelprodukt von geringem Wert anfällt, eine Abtrennung von Brassicasterol wird nicht beschrieben.

Weiterhin ist im Stand der Technik die vollständige Hydrierung von Sterolen bei der Stanoldarstellung bekannt. Insbesondere bei Verwendung von Palladium-Katalysatoren werden dabei sowohl die Doppelbindung zwischen den Kohlenstoffatomen 5 und 6 in der Ringstruktur des Sterols als auch die bei Brassicasterol und Stigmasterol vorhandene Doppelbindung zwischen den Kohlenstoffatomen 22 und 23 der Seitenkette vollständig gesättigt, und es bilden sich die entsprechenden Stanole (Stigmastanol, Campestanol). Selbst eine Teilhydrierung unter milden Bedingungen führt nach dem Stand der Technik immer zu einer Begünstigung der Stanolbildung (siehe auch Modeling and Scale up of Sitosterol Hydrogenation Process, Ind. Eng. Chem. Res. 2006,45,7067-7076), was insbesondere im Hinblick auf die Umsetzung von Campesterol aufgrund der SCF-Limitierung von Campestanol auf maximal 5 Gewichts-% nachteilig ist. Es ist somit nicht möglich, mit einer Hydrierung insbesondere von rapsstämmigen Phytosterol-Phytostanolzusammensetzungen nach dem Stand der Technik eine Phytosterol-Phytostanolzusammensetzung zu erhalten, welche den Vorgaben des SCF entspricht.

JP 2001 335795 A offenbart ein Verfahren zur Herstellung einer Phytosterol-Phytostanolzusammensetzung, wobei das bei der Hydrierung eingesetzte Edukt 1,8 % Brassicasterol umfasst und zur Hydrierung ein Raney-Nickel-Katalysator verwendet wird.

DE 696 32 481 T2 offenbart die vollständige Hydrierung einer Sterolmischung, die 2,7 % Brassicasterol umfasst, zu Stanolen. Verwendet wird ein Palladium-Katalysator.

Aus der Literatur sind Verfahren bekannt, die eine temporäre Maskierung der zwischen den Kohlenstoffatomen 5 und 6 in der Ringstruktur des Sterols vorhandenen Doppelbindungen über Cyclopropyl-Methylether oder Epoxidierung zur Hydrierung ausschließlich der Doppelbindungen in den Sterol-Seitenketten beschreiben, mit dem Ziel die Stanolbildung zu unterdrücken (siehe auch: Hang, Jiliang, "I. Synthesis of β-Sitosterol and Phytosterol Esters; II. New Methodology for Singlet Oxygen Generation from 1,1-Dihydroperoxides" (2012). Student Research Projects, Dissertations, and Theses - Chemistry Department. Paper 31,Seiten 4 und 15ff; University of Nebraska-Lincoln, und: Hang, Jiliang and Dussault, Patrick, "A concise synthesis of β-sitosterol and other phytosterols" (2010). Patrick Dussault Publications. Paper 14, University of Nebraska-Lincoln). Nachteil dieser Verfahren ist aber, das einerseits zusätzliche, verfahrenstechnisch aufwändige Prozessschritte notwendig werden und andererseits unerwünschte Nebenreaktionsprodukte entstehen, was im Hinblick auf eine Verwendung der Produkte als Lebensmittelzusatzstoff aber auch aus ökonomischer Sicht ungünstig ist.

Weiterhin ist im Stand der Technik ein Verfahren zur Reduktion von Brassicasterol zu 22,23-Dihydrobrassicasterol bekannt. So beschreiben Kircher et al. in Lipids, 1973, Vol. 8, Nr. 8, Seiten 453-458 die Hydrierung von aufwändig gereinigtem Brassicasterol zu 22,23-Dihydrobrassicasterol. Gemäß der Lehre dieses Dokuments ist die Verfügbarkeit eines reinen 22,23-Dihydrobrassicasterols Motivation für die direkte Hydrierung des Brassicasterols. Offenbart wird, dass aus einem Dämpferdestillat gewonnenes Brassicasterol durch mehrere Verfahrensschritte, nämlich Acetylierung, Bromierung und anschließende chromatographische Trennung gereinigt und dann unter Verwendung von Raney-Nickel-Katalysator (> 100 Gewichts-% bezogen auf die Steroleinwaage) und Ethylacetat als Lösemittel (< 1 Gewichts-% Sterole in der Lösung) hydriert wird. Die Bildung von Ergostanol als Nebenreaktionsprodukt wird beschrieben. Nachteilig ist bei dem offenbarten Verfahren, dass eine aufwendige Reinigung und Abtrennung des Brassicasterols von den anderen Komponenten des Dämpferdestillats erfolgen muss. Die im Dokument beschriebenen Reaktionsbedingungen, insbesondere die verwendeten großen Mengen an Katalysator und Lösemittel, sind im Hinblick auf eine technisch ökonomische Nutzung des Verfahrens ungeeignet. Das Dokument offenbart nicht die selektive Hydrierung eines Phytostanol-Phytosterolkonzentrats, in welchem Brassicasterol neben weiteren Phytosterolen und Phytostanolen enthalten ist. Das Dokument gibt auch keinen Hinweis darauf, dass eine Phytosterol-Phytostanolzusammensetzung derart selektiv hydriert werden kann, dass eine Zusammensetzung erhalten wird, welche die Vorgaben des SCF erfüllt.

Patentschrift DE 959186 offenbart ein Verfahren, in welchem Ergosterol bzw. dessen Acylderivate unter Verwendung von Raney-Nickel-Katalysator selektiv zu (3β)-Ergosta-7,22-dien-3-ol hydriert wird. Als geeignete Lösemittel werden aromatische wie aliphatische Kohlenwasserstoffe, niedrigmolekulare aliphatische Alkohole und Dioxan genannt, die Hydrierung findet vorzugsweise bei Drucken bis 4 bar und Temperaturen von 20 bis 40 °C statt, die Anwendung schließt höhere Drucke und Temperaturen aber ausdrücklich nicht aus. Gemäß der Offenbarung der DE 959186 liegt die Katalysatordosierung unter 30 Gewichts-% bezogen auf den Steroleinsatz und es befinden sich bis über 10 Gewichts-% Ergosterol(derivate) in der Reaktionslösung. Ergosterol unterscheidet sich zum Brassicasterol lediglich im Vorhandensein einer weiteren Doppelbindung zwischen den Kohlenstoffatomen 7 und 8 in der Ringstruktur des Sterols, der Aufbau der Seitenketten einschließlich der Doppelbindung zwischen den Kohlenstoffatomen 22 und 23 ist identisch. Es ist bemerkenswert, dass es unter den beanspruchten Reaktionsbedingungen und der Verwendung von Raney-Nickel als Katalysator zu keiner Hydrierung der Doppelbindung in der Seitenkette kommt. Auch dieses Dokument gibt keinen Hinweis darauf, dass eine Phytosterol-Phytostanolzusammensetzung derart selektiv hydriert werden kann, dass eine Zusammensetzung erhalten wird, welche die Vorgaben des SCF erfüllt.

In einer weiteren Veröffentlichung des Department of Agricultural Biochemistry, The University of Arizona wird von Versuchen zur selektiven Hydrierung von Stigmasterol zu β-Sitosterol (Kircher et al. in Lipids, 1973, Vol. 8, Nr. 3, Seiten 101-106) auf Basis von Raney-Nickel-Katalysator unter Reaktionsbedingungen ähnlich denen des oben zitierten Verfahrens zur 22,23-Dihydrobrassicasterol-Gewinnung berichtet. Die Schrift offenbart, dass auch bei milden Reaktionsbedingungen eine hohe Tendenz zur Bildung der entsprechenden Stanole bei Raney-Nickel katalysierten Hydrierreaktionen vorliegt. Zu analogen Erkenntnissen kommen auch jüngere Veröffentlichungen (siehe auch: Hang, Jiliang, "I. Synthesis of β-Sitosterol and Phytosterol Esters; II. New Methodology for Singlet Oxygen Generation from 1,1-Dihydroperoxides" (2012). Student Research Projects, Dissertations, and Theses - Chemistry Department. Paper 31, Seite 5f, University of Nebraska-Lincoln; und auch: M. Miesch et al., Synthesis of highly pure oxyphytosterols and (oxy)phytosterol esters, Part I. Regioselective hydrogenation of stigmasterol: An easy access to oxyphytotosterols, Steroids 73(2008), Seiten 702-707). Auch keines dieser Dokumente gibt einen Hinweis darauf, dass eine Phytosterol-Phytostanolzusammensetzung derart selektiv hydriert werden kann, dass eine Zusammensetzung erhalten wird, welche die Vorgaben des SCF erfüllt.

Um rapsölstämmige Phytosterol-Phytostanolkonzentrate, wie sie beispielsweise als Nebenprodukt der Biodieselproduktion gewonnen werden können, auch unter ökonomischen Gesichtspunkten in einem den Vorgaben des SCF entsprechenden Spektrum zur Bereitstellung als Lebensmittelzusatzstoff nutzbar zu machen, ist es notwendig, dass auf mehrstufige Verfahren und aufwändige Trennungsverfahren verzichtet werden kann. Wird - wie in der vorliegenden Erfindung - eine Hydrierung durchgeführt, dürfen sämtliche im Edukt enthaltenen Phytosterole nur in einem solchen Maß hydriert werden, dass der Anteil der hydrierten Verbindungen auch noch den Vorgaben des SCF entspricht. Eine Hydrierung zu Stanolen, beispielsweise um den Anteil an Brassicasterol zu senken, darf nur im Rahmen der Vorgaben des SCF erfolgen.
Die reinen Hydrierverfahren, die im Stand der Technik bekannt sind, würden bei Anwendung auf eine rapsölstämmige Eduktzusammensetzung mit dem Ziel der Absenkung des Brassicasterolgehaltes auf unter 3% jedoch dazu führen, dass im Produkt beispielsweise der gemäß SCF Vorgaben zulässige Maximalgehalt an Campestanol von 5 Gewichts-% überschritten wird.
Eine wirtschaftlich vertretbare Nutzung rapsölstämmiger Phytosterol-Phytostanolkonzentrate zur Bereitstellung als Lebensmittelzusatzstoff wäre damit nicht mehr möglich, weil eine Abtrennung der überschüssigen Stanolanteile erfolgen müsste.
Bei einer Eduktzusammensetzung, wie sie in der vorliegenden Erfindung verwendet wird, also unter anderem mit einem Anteil von > 3 Gewichts-% Brassicasterol und 3 bis 40 Gewichts-% Campesterol, sind die im Stand der Technik beschriebenen Verfahren somit nicht anwendbar.

Aufgabe der vorliegenden Erfindung ist es deshalb, die Nachteile des Standes der Technik zu überwinden.

Überraschenderweise haben die Erfinder gefunden, dass unter Verwendung eines Nickel-Hydrierkatalysators die Hydrierung der Eduktzusammensetzung derart erfolgen kann, dass sämtliche im Edukt enthaltene Phytosterole nur in einem Anteil hydriert werden, welcher ein Produkt gemäß der Vorgaben des SCF ergibt.

Aufgabe der vorliegenden Erfindung ist es deshalb, ein Verfahren bereitzustellen, mittels welchem eine Phytosterol-Phytostanolzusammensetzung in einer quantitativen Umsetzung derart modifiziert werden kann, dass sie nach der Modifizierung den Empfehlungen des SCF entspricht, indem sie ≤ 80 Gewichts-% β-Sitosterol, ≤ 15 Gewichts-% Stigmastanol, ≤ 40 Gewichts-% Campesterol, ≤ 5 Gewichts-% Campestanol, ≤ 30 Gewichts-% Stigmasterol, ≤ 3 Gewichts-% Brassicasterol und ≤ 3 Gewichts-% sonstige Phytosterole umfasst.

Erfindungsgemäß wird die Aufgabe gelöst durch die Bereitstellung eines Verfahrens zur Herstellung einer Phytosterol-Phytostanolzusammensetzung, umfassend ≤ 3 Gewichts-% Brassicasterol, ≤ 40 Gewichts-% Campesterol, ≤ 5 Gewichts-% Campestanol, ≤ 80 Gewichts-% β-Sitosterol, ≤ 30 Gewichts-% Stigmasterol, ≤ 15 Gewichts-% Stigmastanol, wobei man in Schritt a) eine Phytosterol-Phytostanol-Eduktzusammensetzung bereitstellt, umfassend > 3 Gewichts-% Brassicasterol, 3 bis 40 Gewichts-% Campesterol, < 5 Gewichts-% Campestanol, ≤ 80 Gewichts-% β-Sitosterol, ≤ 30 Gewichts-% Stigmasterol, < 15 Gewichts-% Stigmastanol,
in einem Schritt b) die Phytosterol-Phytostanol-Eduktzusammensetzung aus Schritt a) in einem Lösemittel löst, in einem Schritt c) die gelöste Phytosterol-Phytostanol-Eduktzusammensetzung aus Schritt b) mittels eines Nickel-Hydrierkatalysators hydriert und in einem Schritt d) die gebildete Phytosterol-Phytostanolzusammensetzung isoliert, und man in Schritt c) die Hydrierung in einem Temperaturbereich von 40 °C bis 120 °C und bei einem Druck von 4.000 hPa bis 50.000 hPa durchführt,
wobei sich die Gewichtsangaben auf die Gesamtmasse an Phytosterolen und Phytostanolen in ihrer freien, nicht-derivatisierten Form beziehen und
wobei die Phytostanole und Phytosterole in den Zusammensetzungen in freier, nicht-derivatisierter Form, als in der 3-Position substituierte Phytosterolderivate und Phytostanolderivate, in ihren epimeren Formen oder als deren Mischungen vorliegen.

Insbesondere vorteilhaft ist ein Verfahren, bei welchem man in Schritt a) eine Phytosterol-Phytostanol-Eduktzusammensetzung bereitstellt, welche ≥ 5 Gewichts-% Brassicasterol, 6 bis 40 Gewichts-% Campesterol, ≤ 4 Gewichts-% Campestanol, ≤ 70 Gewichts-% β-Sitosterol, ≤ 10 Gewichts-% Stigmasterol und ≤ 10 Gewichts-% Stigmastanol umfasst, bezogen auf die Gesamtmasse an Phytosterolen und Phytostanolen in ihrer freien, nicht-derivatisierten Form, wobei die Phytosterole und Phytostanole in freier, nicht-derivatisierter Form, als in der 3-Position substituierte Phytosterolderivate und Phytostanolderivate, in ihren epimeren Formen oder deren Mischungen vorliegen.

Insbesondere vorteilhaft ist ein Verfahren, bei welchem man in Schritt a) eine Phytosterol-Phytostanol-Eduktzusammensetzung bereitstellt, welche
5 bis 15 Gewichts-% Brassicasterol,
6 bis 40 Gewichts-% Campesterol,
≤ 4 Gewichts-% Campestanol,
≤ 70 Gewichts-% β-Sitosterol,
≤ 10 Gewichts-% Stigmasterol und
≤ 10 Gewichts-% Stigmastanol umfasst, bezogen auf die Gesamtmasse an Phytosterolen und Phytostanolen in ihrer freien, nicht-derivatisierten Form, wobei die Phytosterole und Phytostanole in freier, nicht-derivatisierter Form, als in der 3-Position substituierte Phytosterolderivate und Phytostanolderivate, in ihren epimeren Formen oder deren Mischungen vorliegen.

Besonders vorteilhaft ist ein Verfahren, bei welchem man in Schritt a) eine Phytosterol-Phytostanol-Eduktzusammensetzung bereitstellt, umfassend 9 bis 13 Gewichts-% Brassicasterol, 25 bis 40 Gewichts-% Campesterol, ≤ 1 Gewichts-% Campestanol, 40 bis 60 Gewichts-% β-Sitosterol, ≤ 1 Gewichts-% Stigmasterol und ≤ 1 Gewichts-% Stigmastanol, bezogen auf die Gesamtmasse an Phytosterolen und Phytostanolen in ihrer freien, nicht-derivatisierten Form, wobei die Phytosterole und Phytostanole in freier, nicht-derivatisierter Form, als in der 3-Position substituierte Phytosterolderivate und Phytostanolderivate, in ihren epimeren Formen oder deren Mischungen vorliegen.

Erfindungsgemäß bevorzugt ist ein Verfahren, bei welchem die in 3-Position substituierten Phytosterolderivate oder Phytostanolderivate Phytosterolester, Phytostanolester, Phytosterolether, Phytostanolether oder deren Mischungen sind.

Weiterhin vorteilhaft ist ein Verfahren, bei welchem die Phytosterolderivate und Phytostanolderivate Ester kurzkettiger aliphatischer Carbonsäuren oder Ester pflanzenölstämmiger Fettsäuren, oder deren Mischungen sind.

Darüber hinaus bevorzugt ist ein Verfahren, bei welchem die kurzkettigen aliphatischen Carbonsäuren ausgewählt sind aus Ameisensäure, Essigsäure, Buttersäure und Propionsäure.

Vorteilhaft ist ein Verfahren, bei welchem die pflanzenölstämmigen Fettsäuren ausgewählt sind aus Laurinsäure, Myristinsäure, Arachinsäure, Behensäure, Hexadekensäure, Gadoleinsäure, Erucasäure, Ölsäure, Linolsäure, Linolensäure, Palmitinsäure und Stearinsäure.

Weiterhin bevorzugt ist ein Verfahren, bei welchem die Phytosterolderivate und Phytostanolderivate Phytosterolglykoside, Phytostanolglykoside oder deren Mischungen sind.

Auch bevorzugt ist ein Verfahren, bei welchem man in einem weiteren Schritt e) in der Phytosterol-Phytostanolzusammensetzung enthaltene freie, nicht-derivatisierte Phytosterole und Phytostanole in der 3-Position substituiert und in ihre Ester oder Ether überführt.

Vorteilhaft ist auch ein Verfahren, bei welchem man in einem weiteren Schritt f) die in Schritt d) oder e) erhaltene Phytosterol-Phytostanolzusammensetzung mit einer Phytosterol-Phytostanol-Eduktzusammensetzung gemäß der in Schritt a) eingesetzten Phytosterol-Phytostanol-Eduktzusammensetzung blendet.

Insbesondere bevorzugt ist ein Verfahren, bei welchem man in einem Schritt g) in Schritt f) erhaltene freie, nicht-derivatisierte Phytosterole und Phytostanole in der 3-Position substituiert und in ihre Ester oder Ether überführt.

Weiterhin vorteilhaft ist ein Verfahren, bei welchem man in Schritt a) eine Phytosterol-Phytostanol-Eduktzusammensetzung bereitstellt, welche aus Rapsöl, Sojaöl, Tallöl, Maiskeimöl oder deren Mischungen abgeleitet ist.

Auch bevorzugt ist ein Verfahren, bei welchem man in Schritt a) als Phytosterol-Phytostanol-Eduktzusammensetzung ein Phytosterol-Phytostanolkonzentrat, gewonnen aus Dämpferdestillaten der Pflanzenölraffination, Destillationsrückständen der Biodieselherstellung, deren Ester, deren Ether oder deren Mischungen bereitstellt.

Insbesondere vorteilhaft ist ein Verfahren, bei welchem man in Schritt b) die Phytosterol-Phytostanol-Eduktzusammensetzung in einem organischen Lösemittel löst, das ausgewählt ist aus Alkoholen und Carbonsäureestern und deren Mischungen. Bevorzugte Alkohole sind primäre und sekundäre Alkohole wie Ethanol und 2-Propanol. Ein als Lösemittel bevorzugter Carbonsäureester ist Ethylacetat

Weiterhin bevorzugt ist ein Verfahren, bei welchem man in Schritt c) die Hydrierung unter Verwendung eines aktivierten, aluminiumhaltigen Nickel-Katalysators durchführt und der Nickel-Katalysator mit Eisen, Chrom oder Molybdän promotiert oder unpromotiert ist.

Vorteilhaft ist weiterhin ein Verfahren, bei welchem man den Katalysator in Schritt c) in einer Menge von 0,5 Gewichts-% bis 15 Gewichts-%, bezogen auf die Gesamtmasse an Phytosterolen und Phytostanolen in ihrer freien, nicht-derivatisierten Form einsetzt.

Auch bevorzugt ist ein Verfahren, bei welchem man in Schritt c) die Hydrierung in einem Temperaturbereich von 60 °C bis 100 °C und bei einem Druck von 25.000 bis 35.000 hPa durchführt.

Weiterhin offenbart wird die Bereitstellung einer Phytosterol-Phytostanolzusammensetzung, welche mittels des erfindungsgemäßen Verfahrens hergestellt wurde.

Darüber hinaus offenbart wird die Verwendung einer Phytosterol-Phytostanolzusammensetzung als Zusatzstoff in Lebensmitteln, wobei die Zusammensetzung mittels des erfindungsgemäßen Verfahrens hergestellt wurde.

Erfindungsgemäß wird als Edukt eine Phytosterol-Phytostanolzusammensetzung bereitgestellt, welche mehr als 3 Gewichts-% Brassicasterol umfasst. Da rapsölstämmige Phytosterol-Phytostanolkonzentrate üblicherweise mehr als 3 Gewichts-% Brassicasterol umfassen, können rapsölstämmige Eduktzusammensetzungen dem erfindungsgemäßen Verfahren unterworfen werden. Das als Edukt verwendete Phytosterol-Phytostanolkonzentrat kann jedoch auch aus einer anderen pflanzlichen Quelle stammen und auch ein Gemisch aus Phytosterolen und Phytostanolen verschiedener pflanzlicher Quellen sein.

Besonders vorteilhaft ist, dass auch rein rapsölstämmige Phytosterol-Phytostanolkonzentrate mittels des erfindungsgemäßen Verfahrens in eine Zusammensetzung überführt werden können, welche den Empfehlungen des Scientific Committee on Food (SCF) entspricht. Somit ermöglicht das erfindungsgemäße Verfahren die Nutzung von rapsölstämmigen Phytosterol-Phytostanolkonzentraten unter Einhaltung der Vorgaben des SCF, ohne dass ein Blenden mit aus anderen, nicht rapsölstämmigen Pflanzenölen gewonnen Phytosterolen und Phytostanolen, erforderlich ist.

Neben den explizit vom SCF genannten Grenzen des Anteils bestimmter Phytosterole und Phytostanole wird in der SCF-Empfehlung darüber hinaus festgelegt, dass eine Phytosterol-Phytostanolzusammensetzung maximal 3 Gewichts-% "sonstige Sterole" enthalten darf. Auch diese Vorgabe wird erfüllt, wenn das erfindungsgemäße Verfahren durchgeführt wird.

Hierbei ist es besonders vorteilhaft, dass das erfindungsgemäße Verfahren mit vergleichsweise geringem Aufwand durchgeführt werden kann. Insbesondere vorteilhaft ist, dass das erfindungsgemäße Verfahren eine Modifizierung der Edukt-Phytosterol-Phytostanolzusammensetzung in einer quantitativen Umsetzung unter Maximierung der Ausbeute, ohne Anfall von Kopplungsprodukten zur Produkt-Zusammensetzung ermöglicht.

Die Aufgabe der Erfindung wird also durch ein Verfahren gelöst, das eine selektive Hydrierung eines Phytosterol-Phytostanolkonzentrats umfasst.

In einem weiteren, optionalen Schritt kann das Verfahren das Mischen der im Hydrierungsschritt erhaltenen modifizierten Phytosterol-Phytostanolzusammensetzung mit nicht hydriertem Phytosterol-Phytostanolkonzentrat umfassen.

Hierbei ist besonders vorteilhaft, dass die mittels des erfindungsgemäßen Verfahrens gewonnene Phytosterol-Phytostanolzusammensetzung in diesem optionalen Verfahrensschritt mit einer Zusammensetzung gemischt wird, welche aufgrund ihres Phytosterol-Phytostanolspektrums als Edukt in Verfahrensschritt a) bereitgestellt werden kann.

So kann erfindungsgemäß im Mischungsschritt zur hydrierten Phytosterol-Phytostanolzusammensetzung eine Phytosterol-Phytostanolzusammensetzung als Mischungskomponente zugesetzt werden, die rapsölstämmig ist und nicht vorab durch Hydrierung modifiziert wurde.

Als weitere optionale Schritte werden Verfahrensschritte zur Derivatisierung durchgeführt.

Weiterhin die Bereitstellung der mittels des erfindungsgemäßen Verfahrens hergestellten Phytosterol-Phytostanolzusammensetzung und deren Verwendung als Zusatzstoff in Lebensmitteln offenbart.

Die Erfindung wird nachfolgend näher erläutert.

Unter selektiver Hydrierung wird im Zusammenhang der vorliegenden Erfindung eine Hydrierung verstanden, bei welcher nicht sämtliche, in den Phytosterolen enthaltenen Doppelbindungen hydriert werden.

Überraschend wurde nun gefunden, dass mittels des erfindungsgemäßen Verfahrens bei Durchführung des Verfahrensschritts der selektiven Hydrierung das Spektrum eines Phytosterol-Phytostanolkonzentrats derart verändert werden kann, dass sich der Gehalt an Brassicasterol unter 3 Gewichts-% absenken lässt, ohne dass ein Campestanolgehalt von 5 Gewichts-% überschritten wird. Dieser Verfahrensschritt wird unter Verwendung von Nickel-Katalysatoren durchgeführt.

Geeignete Nickel-Katalysatoren sind insbesondere aktivierte Nickel-Katalysatoren, wie sie zum Beispiel unter den Bezeichnungen "Raney"® - Nickel oder "Sponge Metal"™ im Handel von verschiedenen Herstellern angeboten werden. Diese Katalysatoren werden aus Nickel-AluminiumLegierungen hergestellt und umfassen üblicherweise einen bestimmten Anteil Aluminium. Sie zeichnen sich durch eine aktivierte Metalloberfläche aus, auf welcher bzw. in welcher Wasserstoff adsorbiert ist.

Die geeigneten Nickel-Katalysatoren können auch promotiert sein, wobei die promotierten Nickel-Katalysatoren ausgewählt sind aus Nickel-Katalysatoren, die mit Eisen, Chrom oder Molybdän promotiert sind.

Überraschenderweise gelingt es somit mittels des erfindungsgemäßen Verfahrens, dass auch ein rapsölstämmiges Phytosterol-Phytostanolkonzentrat derart modifiziert werden kann, dass die Vorgaben des SCF erfüllt werden können.

Erfindungsgemäß können als Eduktzusammensetzung Rückstände aus der Aufbereitung und Weiterverarbeitung von Naturprodukten wie Pflanzenölen verwendet werden. Dies ist vorteilhaft, weil derartige Rückstände in vielen Prozessen, wie bei der Biodieselherstellung, in großen Mengen anfallen und dann wertvoll sind, wenn sie kostengünstig für die Gewinnung hochwertiger Produkte eingesetzt werden können.

Bevorzugt ist deshalb das erfindungsgemäß zu modifizierende, also als Edukt verwendete Phytosterol-Phytostanolkonzentrat ein Naturprodukt. Bei der Verwendung eines Naturprodukts muss jedoch berücksichtigt werden, dass dessen Zusammensetzung innerhalb bestimmter Grenzen, die für die pflanzliche Quelle charakteristisch sind, schwanken kann.

Um eine Phytosterol-Phytostanolzusammensetzung gemäß den SCF-Vorgaben mittels des erfindungsgemäßen Verfahrens zu erhalten, kann aufgrund dieser Schwankungen in der Eduktzusammensetzung je nach Charge eine Anpassung der Reaktionsbedingungen der Hydrierung erforderlich werden.

Diese Anpassung kann durchgeführt werden, ist jedoch mit einem entsprechenden Zeitaufwand verbunden. Da vorgesehen ist, das Verfahren in größerem Maßstab kostengünstig durchzuführen, kann erfindungsgemäß der optionale, weitere Verfahrensschritt des Mischens durchgeführt werden. Dieser weitere Verfahrensschritt ermöglicht es, dass die Reaktionsbedingungen der Hydrierung nicht bei jedem Chargenwechsel angepasst werden müssen. Auf diesem Weg kann das Verfahren kostengünstig, mit verhältnismäßig geringem technischen Aufwand auch in größerem Maßstab durchgeführt werden.

Dieser zusätzliche Verfahrensschritt ist ein Mischungsschritt, bei welchem die bei der Hydrierung erhaltene modifizierte Phytosterol-Phytostanolzusammensetzung mit einem Phytosterol-Phytostanolkonzentrat gemischt wird. Dieses im Mischungsschritt hinzugefügte Phytosterol-Phytostanolkonzentrat besitzt eine Zusammensetzung in Bezug auf die Komponenten und Mengenverhältnisse, wie die in Schritt a) bereitgestellte Zusammensetzung. Jedoch können beide Zusammensetzungen, innerhalb der definierten Mengenverhältnisse, voneinander abweichen. Erfindungsgemäß könnte die im Mischungsschritt hinzugefügte Phytosterol-Phytostanolzusammensetzung auch in Schritt a) des Verfahrens eingesetzt werden.

Das Edukt-Phytosterol-Phytostanolkonzentrat umfasst entsprechend > 3 Gewichts-% Brassicasterol, 3 bis 40 Gewichts-% Campesterol, < 5 Gewichts-% Campestanol, ≤ 80 Gewichts-% β-Sitosterol, ≤ 30 Gewichts-% Stigmasterol, < 15 Gewichts-% Stigmastanol, bezogen auf die Gesamtmasse an Phytosterolen und Phytostanolen in ihrer freien, nicht-derivatisierten Form. Aufgrund dieses Phytosterol-Phytostanolspektrums ist es möglich, auch im Mischungsschritt eine rapsölstämmige Zusammensetzung zu verwenden.

Die Komponenten der Phytosterol-Phytostanolzusammensetzung, die im Mischungsschritt der aus der Hydrierung erhaltenen Phytosterol-Phytostanolzusammensetzung hinzugefügt wird und deren Anteile können mit denen der bei der Hydrierung eingesetzten Edukt-Phytosterol-Phytostanolzusammensetzung identisch sein, können jedoch auch von diesen abweichen. Eine identische Zusammensetzung wird beispielsweise dann vorliegen, wenn ein Teil einer Charge eines Phytosterol-Phytostanolkonzentrats gemäß dem erfindungsgemäßen Verfahren hydriert wird und ein Teil der Charge nicht der Hydrierung unterworfen wird und somit im Mischungsschritt verwendet werden kann. Die Phytosterole und Phytostanole der im Mischungsschritt hinzugefügten Phytosterol-Phytostanolzusammensetzung können in der freien, nicht-derivatisierten Form, als in der 3-Position substituierte Phytosterolderivate und Phytostanolderivate, in ihren epimeren Formen oder als deren Mischungen vorliegen.

Dieses Ausführungsbeispiel des erfindungsgemäßen Verfahrens ist insbesondere vorteilhaft, weil sowohl hydriertes als auch nicht hydriertes Phytosterol-Phytostanolkonzentrat bei der Herstellung der erfindungsgemäßen Zusammensetzung verwendet werden kann. Es genügt somit, dass nur ein Teil der in der späteren Endzusammensetzung enthaltenen Komponenten aus einer selektiven Hydrierung erhalten wurde. Dies senkt die Verfahrenskosten erheblich und macht Phytosterol-Phytostanolkonzentrate mit unterschiedlichen Gehalten an Brassicasterol und Campesterol erfindungsgemäß nutzbar.

In einem Ausführungsbeispiel des erfindungsgemäßen Verfahrens werden Ester oder Ether der Phytosterole und Phytostanole hergestellt. Dazu erfolgt die Veresterung oder Etherbildung als weiterer Prozessschritt entweder vor oder im Anschluss an den Verfahrensschritt der selektiven Hydrierung oder im Anschluss an den Mischungsschritt.

Eine der selektiven Hydrierung nachgeschaltete Derivatisierung hat den Vorteil, dass nicht auch die an das Phytosterol- oder Phytostanolgerüst gebundenen Reste, wie beispielsweise Fettsäurereste bei Vorliegen von Fettsäureestern, ebenfalls hydriert werden. Eine Hydrierung der Fettsäurereste von Phytosterol- und Phytostanolfettsäureestern kann den Schmelzpunkt der Phytosterol- und Phytostanolester unter Umständen unerwünscht erhöhen. Verfahren zur Darstellung von Fettsäureestern der Phytosterole und Phytostanole werden z. B. in der WO 92/19640 oder der EP 0 982 315 offenbart.

Nachfolgend werden die Schritte des erfindungsgemäßen Verfahrens weiter erläutert.

Das erfindungsgemäße Verfahren umfasst im ersten Schritt die Bereitstellung einer Phytosterol-Phytostanolzusammensetzung, die in einem geeigneten Lösemittel in Lösung gebracht wird. Die gelöste Phytosterol-Phytostanolzusammensetzung wird unter milden Reaktionsbedingungen unter Verwendung eines Nickel-Katalysators hydriert. Nach dem Abtrennen des Katalysators (z. B. mittels Filtration) wird die erfindungsgemäße, modifizierte Phytosterol-Phytostanolzusammensetzung durch Abdampfen des Lösemittels isoliert. Optional schließt sich ein Derivatisierungsschritt an, bei welchem die Phytostanole und Phytosterole in der 3-Position substituiert werden.

Gegebenenfalls erfolgt ein weiterer, optionaler Verfahrensschritt, bei welchem die modifizierte Phytosterol-Phytostanolzusammensetzung mit einem Phytosterol-Phytostanolkonzentrat gemischt wird, das die Zusammensetzung des als Edukt eingesetzten Phytosterol-Phytostanolkonzentrats besitzt. Dieser Verfahrensschritt kann direkt im Anschluss an die Isolierung der modifizierten Phytosterol-Phytostanolzusammensetzung erfolgen. Er kann jedoch auch nach der optionalen Derivatisierung durchgeführt werden.

Die Herausforderung bei der Modifizierung der im erfindungsgemäßen Verfahren eingesetzten Edukt-Phytosterol-Phytostanolzusammensetzung, die zum Beispiel eine rapsölstämmige Zusammensetzung sein kann, liegt im Sterol-Stanolspektrum begründet, wenn es gilt, die SCF-Empfehlungen für die Novel-Food-Zulassung einzuhalten.

Neben der Reduktion des Brassicasterolgehalts sollten bei der Modifizierung des Phytosterol-Phytostanolspektrums bevorzugt gleichzeitig die weiteren Grenzwerte der SCF-Empfehlung nicht überschritten werden. Aus diesem Grund darf der Gehalt an Campesterol nicht über 40 Gewichts-% ansteigen. Es muss deshalb ein Verfahren zur Verfügung gestellt werden, bei welchem parallel zur Hydrierung des Brassicasterols anteilig auch Campesterol zu Campestanol umgewandelt wird. Hierbei muss jedoch das Verfahren auch die Bedingung erfüllen, dass der Grenzwert von maximal 5 Gewichts-% Campestanol eingehalten wird.

Überraschenderweise wurde gefunden, dass die erfindungsgemäße Hydrierung einer Phytosterol-Phytostanolzusammensetzung, welche > 3 Gewichts-% Brassicasterol, 3 bis 40 Gewichts-% Campesterol, < 5 Gewichts-% Campestanol, ≤ 80 Gewichts-% β-Sitosterol, ≤ 30 Gewichts-% Stigmasterol und < 15 Gewichts-% Stigmastanol umfasst, zu einer Phytosterol-Phytostanolzusammensetzung führt, welche den Vorgaben des SCF entspricht. Erfindungsgemäß erfolgt die Hydrierung unter Verwendung eines Nickel-Katalysators.

Es wurden Versuchsreihen an einem Labor-Hydrierautoklaven durchgeführt, bei welchen Reaktionszeit, Reaktionstemperatur, Rührerdrehzahl, Wasserstoffdruck und Katalysator sowie die Katalysatordosierung variiert wurden.

Im Verfahrensschritt a) wird eine Phytosterol-Phytostanolzusammensetzung bereitgestellt. Diese Zusammensetzung kann beispielsweise eine rapsölstämmige Zusammensetzung sein. Ein solche Zusammensetzung ist beispielsweise nach dem in der EP 2 635 592 offenbarten Verfahren aus der Rapsöl-Methylesterproduktion erhältlich.

Diese Phytosterol-Phytostanolzusammensetzung wird in Schritt b) des Verfahrens in einem geeigneten Lösemittel gelöst. Geeignete Lösemittel sind beispielsweise Ethanol, Ethylacetat und 2-Propanol. Die Konzentration an Phytosterolen und Phytostanolen in der Lösung beträgt erfindungsgemäß 5 bis 50 Gewichts-%, vorzugsweise 10 bis 40 Gewichts-%.

Im Verfahrensschritt c) erfolgt die selektive Hydrierung der Phytosterol-Phytostanolzusammensetzung. Diese selektive Hydrierung erfolgt unter Verwendung eines Nickel-Katalysators, der ausgewählt ist aus mit Eisen, Chrom oder Molybdän promotierten Nickel-Katalysatoren und Raney-Nickel.

Die Dosierung des Katalysators beträgt 0,5 bis 15 Gewichts-%, bevorzugt 2 bis 6 Gewichts-% bezogen auf die Gesamtmasse an Phytosterol und Phytostanol in ihrer freien, nicht-derivatisierten Form.

Der spezifische Bedarf an Frischkatalysator lässt sich durch die Wiederverwendung des gebrauchten Katalysators über mehrere Prozesszyklen deutlich absenken. Hierbei ist unter Frischkatalysator ein Katalysator zu verstehen, der noch nicht zur Hydrierung verwendet wurde. Gebrauchter Katalysator wurde bereits in mindestens einem Reaktionszyklus der Hydrierung eingesetzt und anschließend zurückgewonnen.

Erfindungsgemäß wird die Hydrierung bei einer Temperatur von 40 °C bis 120 °C durchgeführt. Bevorzugt wird die Hydrierung in einem Temperaturbereich zwischen 60 °C und 100 °C durchgeführt. Die Reaktionszeit liegt in einem bevorzugten Ausführungsbeispiel im Bereich von 1 bis 2 Stunden.

Erfindungsgemäß wird der Verfahrensschritt der Hydrierung bei einem Wasserstoffdruck von 4000 hPa bis 50.000 hPa und insbesondere bevorzugt bei einem Wasserstoffdruck von 25.000 bis 35.000 hPa durchgeführt.

Im Anschluss an die Hydrierung wird im Schritt d) des Verfahrens die modifizierte Phytosterol-Phytostanolzusammensetzung isoliert.

Die in Schritt d) des Verfahrens erhaltene, modifizierte Phytosterol-Phytostanolzusammensetzung kann einem weiteren Verfahrensschritt f) unterzogen werden. In diesem Reaktionsschritt wird ein bestimmter Anteil modifizierte Phytosterol-Phytostanolzusammensetzung aus Schritt d) mit einem bestimmten Anteil Phytosterol-Phytostanolkonzentrat vermischt, welches eine Phytosterol-Phytostanolzusammensetzung besitzt wie die in Schritt a) bereitgestellte Zusammensetzung. Dies wird in Beispiel 2 näher spezifiziert.

Weiterhin können die in Schritt d) und Schritt f) erhaltenen Phytosterole und Phytostanole derivatisiert werden. Hierzu umfasst das erfindungsgemäße Verfahren die Verfahrensschritte e) und g).

Die nachfolgenden Beispiele erläutern die Erfindung näher, ohne jedoch deren Schutzumfang zu beschränken.

Die Beispiele 1 und 2 sind Ausführungsbeispiele des erfindungsgemäßen Verfahrens. In Beispiel 1 wird ein Ausführungsbeispiel des erfindungsgemäßen Verfahrens offenbart, bei welchem die Verfahrensschritte a) bis d) durchgeführt werden. Bei dem in Beispiel 2 offenbarten Ausführungsbeispiel wird zusätzlich zu den Verfahrensschritten a) bis d) noch der Mischungsschritt f) durchgeführt.

Die nachfolgenden Beispiele veranschaulichen die Erfindung, ohne diese jedoch einzuschränken. In den nachfolgenden Beispielen wird eine rapsölstämmige Zusammensetzung eingesetzt. Das erfindungsgemäße Verfahren kann jedoch auch mit Phytosterol-Phytostanolzusammensetzungen aus anderen pflanzlichen Quellen und deren Mischung ausgeführt werden.

### Beispiel 1

Es wurden 5 g eines rapsölstämmigen Phytosterol-Phytostanolkonzentrats (Charge T6) bereitgestellt. Dieses Konzentrat wurde aus dem Destillationsrückstand aus der Rapsöl-Methylester-Produktion entsprechend der Lehre der EP 2 635 592 erhalten. Das Konzentrat besaß einen Anteil von 95,3 Gewichts-% Phytosterole und Phytostanole, in ihrer freien, nicht-derivatisierten Form. Das rapsölstämmige Phytosterol-Phytostanolkonzentrat wurde in einen 100ml-Labor-Hydrierautoklaven (Büchi Typ Midiclave, mit Begasungsrührwerk) zusammen mit 60 ml 2-Propanol und 0,6 g aktiviertem Nickel-Katalysator ("Sponge Metal Catalyst", Typ A 5B00, Johnson Matthey, 6 % Aluminium, mittlere Teilchengröße 50 Mikron) gegeben.
Der Reaktor wurde verschlossen, mit Stickstoff inertisiert und unter Rühren aufgeheizt. Nach Erreichen der Reaktionstemperatur von 60 °C wurde der Reaktor mit Wasserstoff bis zu einem Druck von 28.000 hPa beaufschlagt. Nach Ablauf einer Reaktionszeit von 1 Stunde wurde der Autoklav entspannt und mit Stickstoff inertisiert. Nach dem Stoppen des Rührwerkes sedimentierte der Raney-Nickel-Katalysator auf den Boden des Reaktionsgefäßes ab. Die überstehende Reaktionslösung wurde mittels einer Spritze (mit Spritzenfilter 1 µm) abgezogen und am Rotationsverdampfer eingeengt. Es resultierte ein Produkt mit einem Sterol-Stanolgehalt von 95,9 Gewichts-%, bezogen auf die eingeengte Gesamtprobe. Die Zusammensetzung des Reaktionsprodukts, in Bezug auf die enthaltenen Sterole und Stanole ist in Tabelle 1 gezeigt. Das Reaktionsprodukt ist mit SH34 bezeichnet.

Tabelle 1 zeigt die Sterol-Stanol-Zusammensetzung des in Beispiel 1 als Edukt eingesetzten Phytosterol-Phytostanolkonzentrats, das mit Charge T6 bezeichnet wird. Aufgeführt wird weiterhin die Sterol-Stanol-Zusammensetzung des Produkts aus Beispiel 1 (SH34). Diesen Werten wird die SCF-Empfehlung gegenübergestellt.

**Tabelle 1: Phytosterol-Phytostanolzusammensetzung aus Beispiel 1**

| Zusammensetzung | Edukt Charge T6 *[Gewichts-%] | Produkt SH34 *[Gewichts-%] | SCF-Empfehlung *[Gewichts-%] |
|---|---|---|---|
| Cholesterol | 0,2 | 0,2 | |
| Brassicasterol | 10,4 | 2,9 | ≤ 3 |
| Campesterol | 35,5 | 40,0 | ≤ 40 |
| Campestanol | 0,6 | 3,1 | ≤ 5 |
| β-Sitosterol | 48,1 | 47,7 | ≤ 80 |
| Stigmasterol | 0,3 | 0,1 | ≤ 30 |
| Stigmastanol | 0,6 | 3,0 | ≤ 15 |
| sonstige Sterole | 4,3 | 3,0 | ≤ 3 |
| Σ Sterolgehalt | 100,0 | 100,0 | |

| | | | |
|---|---|---|---|
| * Gewichts-% bezogen auf die Gesamtmasse an Phytosterolen und Phytostanolen in ihrer freien, nicht-derivatisierten Form. | | | |

Aufgrund des hohen, aber schwankenden Brassicasterol- und Campesterolgehalts in den als Edukt eingesetzten Phytosterol-Phytostanolkonzentraten, kann eine Anpassung der Reaktionsparameter des erfindungsgemäßen Verfahrens bei jedem Chargenwechsel des Edukts erforderlich sein, um eine Phytosterol-Phytostanolzusammensetzung gemäß den SCF-Vorgaben mittels des erfindungsgemäßen Verfahrens zu erhalten. Dies ist möglich, jedoch mit einem entsprechenden Zeitaufwand und damit auch Kosten verbunden.

Wie bereits erläutert, umfasst das erfindungsgemäße Verfahren in einem Ausführungsbeispiel einen optionalen Schritt f), der sich an die Verfahrensschritte a) bis d) oder a) bis e) anschließt.

In Verfahrensschritt f) wird eine im Schritt d) oder Schritt e) erhaltene, modifizierte Phytosterol-Phytostanolzusammensetzung mit einem Phytosterol-Phytostanolkonzentrat gemischt, welches in Bezug auf seine Bestandteile und Mengenverhältnisse dieser Bestandteile im Bereich der Zusammensetzung liegt, die auch in Verfahrensschritt a) eingesetzt wird.

Wird das erfindungsgemäße Verfahren mit dem zusätzlichen Verfahrensschritt f), also dem Mischungsschritt durchgeführt, so wird Schritt c) bevorzugt so geführt, dass eine möglichst vollständige Reduktion des Brassicasterols erfolgt, ohne dass der Gehalt an Campesterol über 42 Gewichts-% ansteigt.

Nachfolgend wird als Beispiel 2 ein Ausführungsbeispiel des erfindungsgemäßen Verfahrens aufgeführt, das den optionalen Schritt f) umfasst.

Hierzu zeigt Tabelle 2 die Ergebnisse des Beispiels 2. Neben der Zusammensetzung des als Edukt (Charge V37) eingesetzten Phytosterol-Phytostanolkonzentrats wird die Zusammensetzung der im Schritt d) des erfindungsgemäßen Verfahrens gewonnenen modifizierten Phytosterol-Phytostanolzusammensetzung (SH54) aufgeführt. In Spalte 4 der Tabelle 2 wird die Zusammensetzung einer Phytosterol-Phytostanolzusammensetzung gezeigt, welche aus Schritt f) des erfindungsgemäßen Verfahrens erhalten wurde.

Wie in Tabelle 2 angegeben, wurden im Mischungsschritt f) eine Zusammensetzung mit den Merkmalen der Zusammensetzung verwendet, wie sie auch in dem erfindungsgemäßen Verfahrensschritten a) als Edukt eingesetzt wurde (Charge V37). Die Auswahl dieser Charge als Phytosterol-Phytostanolkonzentrat für den Mischungsschritt ist jedoch nur beispielhaft. Erfindungsgemäß kann in Verfahrensschritt f) auch ein Phytosterol-Phytostanolkonzentrat eingesetzt werden, das sich in seiner Zusammensetzung von der spezifischen Zusammensetzung, die in Schritt a) als Edukt eingesetzt wird, unterscheidet. Wobei die im Mischungsschritt zugesetzte Zusammensetzung jedoch in Bezug auf ihre Bestandteile und Mengenverhältnisse dieser Bestandteile im Bereich der Zusammensetzung liegt, die auch in Verfahrensschritt a) eingesetzt wird.

In Beispiel 2 wurde Charge V37 Phytosterol-Phytostanolkonzentrat im Mischungsschritt mit der modifizierten Zusammensetzung SH54, die in Schritt d) des mit Beispiel 2 gekennzeichneten Ausführungsbeispiels erhalten wurde im Massenverhältnis 1:3 gemischt und das Mischungsprodukt erhalten. Die Zusammensetzung des Mischungsprodukts wird in Spalte 4 von Tabelle 2 aufgeführt und den Vorgaben des SCF gegenübergestellt. Dieser Vergleich zeigt, dass mittels des erfindungsgemäßen Verfahrens eine Phytosterol-Phytostanolzusammensetzung erhalten wird, die den Vorgaben der SCF entspricht.

### Beispiel 2

Es wurden 6,6 g eines rapsölstämmigen Phytosterol-Phytostanolkonzentrats (Charge V37) bereitgestellt. Dieses Konzentrat wurde aus dem Destillationsrückstand aus der Rapsöl-Methylester-Produktion entsprechend der Lehre der EP 2 635 592 erhalten. Das Konzentrat besaß einen Anteil von 98,1 Gewichts-% Phytosterole und Phytostanole, in ihrer freien, nicht-derivatisierten Form. Die rapsölstämmige Phytosterol-Phytostanolzusammensetzung wurde in einen 100ml-Labor-Hydrierautoklaven (Büchi Typ Midiclave, mit Begasungsrührwerk) zusammen mit 80 ml 2-Propanol und 0,2 g aktiviertem Nickel-Katalysator ("Sponge Metal Catalyst", Typ A 5B00, Johnson Matthey, 6 % Aluminium, mittlere Teilchengröße 50 Mikron) gegeben.
Der Reaktor wurde verschlossen, mit Stickstoff inertisiert und unter Rühren aufgeheizt. Nach Erreichen der Reaktionstemperatur von 85 °C wurde der Reaktor mit Wasserstoff bis zu einem Druck von 28.000 hPa beaufschlagt. Nach Ablauf einer Reaktionszeit von 2 Stunden wurde der Autoklav entspannt und mit Stickstoff inertisiert. Nach dem Stoppen des Rührwerkes sedimentierte das Raney-Nickel auf den Boden des Reaktionsgefäßes, die überstehende Reaktionslösung wurde mittels einer Spritze (mit Spritzenfilter 1 µm) abgezogen und am Rotationsverdampfer eingeengt. Es resultierte ein Produkt, mit SH 54 gekennzeichnet, mit einem Phytosterol-Phytostanolgehalt von 96,8 Gewichts-%, bezogen auf die eingeengte Gesamtprobe. Die Zusammensetzung des Reaktionsprodukts, in Bezug auf die enthaltenen Sterole und Stanole ist in Tabelle 2 gezeigt.

**Tabelle 2: Phytosterol-Phytostanolzusammensetzung aus Beispiel 2**

| Zusammensetzung | Edukt Charge V37 *[Gewichts-%] | Produkt SH54 *[Gewichts-%] | Mischungsprodukt Edukt: Produkt (1:3) *[Gewichts-%] | SCF-Empfehlung *[Gewichts-%] |
|---|---|---|---|---|
| Cholesterol | 0,2 | 0,3 | 0,3 | |
| Brassicasterol | 11,7 | 0,1 | 3,0 | ≤ 3 |
| Campesterol | 35,4 | 41,5 | 40,0 | ≤ 40 |
| Campestanol | 0,3 | 5,6 | 4,3 | ≤ 5 |
| β-Sitosterol | 48,1 | 45,8 | 46,3 | ≤ 80 |
| Stigmasterol | 0,4 | 0,0 | 0,1 | ≤ 30 |
| Stigmastanol | 0,1 | 4,8 | 3,6 | ≤ 15 |
| sonstige Sterole | 3,8 | 2,0 | 2,4 | ≤ 3 |
| Σ Sterolgehalt | 100 | 100 | 100 | |

| | | | | |
|---|---|---|---|---|
| * Gewichts-% bezogen auf die Gesamtmasse an Phytosterolen und Phytostanolen in ihrer freien, nicht-derivatisierten Form | | | | |

Wie bereits erläutert, werden im Stand der Technik Analyseverfahren für Phytosterol-Phytostanolzusammensetzungen empfohlen und sind anerkannt. Die Analyse der Phytosterol-Phytostanolzusammensetzungen erfolgte gaschromatographisch, z.B. gemäß *European Official Method of Analysis* in der *Commission Regulation EEC* 2568/1991 im Anhang V (ab 5.3).

Dazu müssen die Phytosterole und Phytostanole als freie Sterole und Stanole vorliegen. So müssen beispielsweise Sterolester und Stanolester durch eine dem Fachmann bekannte Methode in freie Sterole bzw. Stanole überführt werden. Dies geschieht beispielsweise durch die einem Fachmann bekannte Verseifung mit ethanolischer KOH und anschließende Extraktion (*EEC* 2568/1991 im Anhang V).

Vor der gaschromatographischen Analyse werden die freien Sterole und Stanole in die entsprechenden Trimethylsilylether überführt. Entsprechende Verfahren sind dem Fachmann bekannt. So kann die Überführung in die Trimethylsilylether beispielsweise mittels BSTFA (N,O-Bis(trimethylsilyl)-trifluoracetamid) oder, wie in *EEC* 2568/1991 im Anhang V Punkt 5.3 beschrieben, erreicht werden.

Für die gaschromatographische Analyse wird z. B. eine Chromatographiesäule mit einer Länge von 20 bis 30 m, einem Innendurchmesser von 0,25 bis 0,32 mm und einer Filmdicke von 0,1 bis 0,3 µm verwendet. Als stationäre Phase wird eine SE52- oder SE 54-Trennphase benutzt.
SE52 besteht zu 5 % aus Phenyl- und zu 95 % aus Methylpolysiloxan. SE54 besteht zu 5 % aus Phenyl-, zu 1 % aus Vinyl- und zu 94 % aus Methylpolysiloxan.
Die weiteren Parameter sind: 260 °C Ofentemperatur (isotherm), 280 °C Injektortemperatur, 290 °C Detektortemperatur (FID), 20 bis 35 cm/s Helium oder 30 bis 50 cm/s Wasserstoff.

Die vorliegende Erfindung stellt ein Verfahren zur Herstellung einer Phytosterol-Phytostanolzusammensetzungen bereit. Die mittels des Verfahrens hergestellte Zusammensetzung ist dadurch charakterisiert, dass das Spektrum der Phytosterole und Phytostanole einen stark reduzierten Gehalt an Brassicasterol aufweist. Dies macht die erfindungsgemäß erzeugten Zusammensetzungen als Zusatzstoff für Nahrungsmittel geeignet. Das erfindungsgemäße Verfahren zur Herstellung der Zusammensetzungen zeichnet sich durch einen relativ geringen technischen Aufwand bei quantitativem Umsatz des Edukts aus und kann die erfindungsgemäßen Zusammensetzungen kostengünstig bereitstellen. Erfindungsgemäß können als Edukt im Verfahren auch rapsölstämmige Phytosterol-Phytostanolzusammensetzungen verwendet werden.

## Patentansprüche

1. Verfahren zur Herstellung einer Phytosterol-Phytostanolzusammensetzung, umfassend
≤ 3 Gewichts-% Brassicasterol,
≤ 40 Gewichts-% Campesterol,
≤ 5 Gewichts-% Campestanol,
≤ 80 Gewichts-% β-Sitosterol,
≤ 30 Gewichts-% Stigmasterol,
≤ 15 Gewichts-% Stigmastanol und
≤ 3 Gewichtsprozent sonstige Sterole,
wobei man
a) eine Phytosterol-Phytostanol-Eduktzusammensetzung bereitstellt, umfassend
> 3 Gewichts-% Brassicasterol,
3 bis 40 Gewichts-% Campesterol,
< 5 Gewichts-% Campestanol,
≤ 80 Gewichts-% β-Sitosterol,
≤ 30 Gewichts-% Stigmasterol,
< 15 Gewichts-% Stigmastanol,
b) die Phytosterol-Phytostanol-Eduktzusammensetzung aus a) in einem Lösemittel löst,
c) die gelöste Phytosterol-Phytostanol-Eduktzusammensetzung aus b) unter Verwendung eines Nickel-Hydrierkatalysators in einem Temperaturbereich von 40 °C bis 120 °C und bei einem Druck von 4.000 hPa bis 50.000 hPa hydriert und
d) die gebildete Phytosterol-Phytostanolzusammensetzung isoliert,
wobei sich die Gewichtsangaben auf die Gesamtmasse an Phytosterolen und Phytostanolen in ihrer freien, nicht-derivatisierten Form beziehen und
wobei die Phytostanole und Phytosterole in den Zusammensetzungen in freier, nicht-derivatisierter Form, als in der 3-Position substituierte Phytosterolderivate und Phytostanolderivate, in ihren epimeren Formen oder als deren Mischungen vorliegen.

2. Verfahren gemäß Anspruch 1, **dadurch gekennzeichnet, dass** man in Schritt a) eine Phytosterol-Phytostanol-Eduktzusammensetzung bereitstellt,
umfassend
≥ 5 Gewichts-% Brassicasterol,
6 bis 40 Gewichts-% Campesterol,
≤ 4 Gewichts-% Campestanol,
≤ 70 Gewichts-% β-Sitosterol,
≤ 10 Gewichts-% Stigmasterol und
≤ 10 Gewichts-% Stigmastanol,
wobei sich die Gewichtsangaben auf die Gesamtmasse an Phytosterolen und Phytostanolen in ihrer freien, nicht-derivatisierten Form beziehen und
wobei die Phytostanole und Phytosterole in der Zusammensetzung in freier, nicht-derivatisierter Form, als in der 3-Position substituierte Phytosterolderivate und Phytostanolderivate, in ihren epimeren Formen oder als deren Mischungen vorliegen.

3. Verfahren gemäß Anspruch einem der vorangehenden Ansprüche, **dadurch gekennzeichnet, dass** man in Schritt a) eine Phytosterol-Phytostanol-Eduktzusammensetzung bereitstellt, umfassend
9 bis 13 Gewichts-% Brassicasterol,
25 bis 40 Gewichts-% Campesterol,
≤ 1 Gewichts-% Campestanol,
40 bis 60 Gewichts-% β-Sitosterol,
≤ 1 Gewichts-% Stigmasterol und
≤ 1 Gewichts-% Stigmastanol,
bezogen auf die Gesamtmasse an Phytosterolen und Phytostanolen in ihrer freien, nicht-derivatisierten Form, wobei die Phytosterole und Phytostanole in freier, nicht-derivatisierter Form, als in der 3-Position substituierte Phytosterolderivate und Phytostanolderivate, in ihren epimeren Formen oder deren Mischungen vorliegen.

4. Verfahren gemäß einem der vorangehenden Ansprüche, **dadurch gekennzeichnet, dass** die in 3-Position substituierten Phytosterolderivate oder Phytostanolderivate Phytosterolester, Phytostanolester, Phytosterolether, Phytostanolether oder deren Mischungen sind.

5. Verfahren gemäß einem der vorangehenden Ansprüche, **dadurch gekennzeichnet, dass** die Phytosterolderivate und Phytostanolderivate Ester kurzkettiger aliphatischer Carbonsäuren oder Ester pflanzenölstämmiger Fettsäuren, oder deren Mischungen sind.

6. Verfahren gemäß einem der vorangehenden Ansprüche, **dadurch gekennzeichnet, dass** die Phytosterolderivate und Phytostanolderivate Phytosterolglykoside, Phytostanolglykoside oder deren Mischungen sind.

7. Verfahren gemäß einem der vorangehenden Ansprüche, **dadurch gekennzeichnet, dass** man in einem weiteren Schritt e) in der Phytosterol-Phytostanolzusammensetzung enthaltene freie, nicht-derivatisierte Phytosterole und Phytostanole in der 3-Position substituiert und in ihre Ester oder Ether überführt.

8. Verfahren gemäß einem der vorangehenden Ansprüche, **dadurch gekennzeichnet, dass** man in einem weiteren Schritt f) die in Schritt d) oder e) erhaltene Phytosterol-Phytostanolzusammensetzung mit einer Phytosterol-Phytostanol-Eduktzusammensetzung gemäß der in Schritt a) eingesetzten Phytosterol-Phytostanol-Eduktzusammensetzung blendet.

9. Verfahren gemäß Anspruch 8, **dadurch gekennzeichnet, dass** man in einem Schritt g) in Schritt f) erhaltene freie, nicht-derivatisierte Phytosterole und Phytostanole in der 3-Position substituiert und in ihre Ester oder Ether überführt.

10. Verfahren gemäß einem der vorangehenden Ansprüche, **dadurch gekennzeichnet, dass** man in Schritt a) als Phytosterol-Phytostanol-Eduktzusammensetzung ein Phytosterol-Phytostanolkonzentrat, gewonnen aus Dämpferdestillaten der Pflanzenölraffination, Destillationsrückständen der Biodieselherstellung, deren Ester, deren Ether oder deren Mischungen bereitstellt.

11. Verfahren gemäß einem der vorangehenden Ansprüche, **dadurch gekennzeichnet, dass** man in Schritt b) die Phytosterol-Phytostanol-Eduktzusammensetzung in einem organischen Lösemittel löst, ausgewählt aus Alkoholen und Carbonsäureestern und deren Mischungen.

12. Verfahren gemäß einem vorangehenden Ansprüche, **dadurch gekennzeichnet, dass** man in Schritt c) die Hydrierung unter Verwendung eines aluminiumhaltigen, aktivierten Nickel-Katalysators durchführt und der Nickel-Katalysator mit Eisen, Chrom oder Molybdän promotiert oder unpromotiert ist.

13. Verfahren gemäß einem der vorangehenden Ansprüche, **dadurch gekennzeichnet, dass** man den Katalysator in Schritt c) in einer Menge von 0,5 Gewichts-% bis 15 Gewichts-%, bezogen auf die Gesamtmasse an Phytosterolen und Phytostanolen in ihrer freien, nicht-derivatisierten Form einsetzt, und/oder man in Schritt c) die Hydrierung in einem Temperaturbereich von 60 °C bis 100 °C und bei einem Druck von 25.000 bis 35.000 hPa durchführt.

## Claims

1. Method for producing a phytosterol-phytostanol composition, comprising
≤ 3 % by weight brassicasterol,
≤ 40 % by weight campesterol,
≤ 5 % by weight campestanol,
≤ 80 % by weight β-sitosterol,
≤ 30 % by weight stigmasterol,
≤ 15 % by weight stigmastanol and
≤ 3 % by weight other sterols,
wherein one
a) provides a phytosterol-phytostanol feed-stock composition comprising
> 3 % by weight brassicasterol,
3 to 40 % by weight campesterol,
< 5 % by weight campestanol,
≤ 80 % by weight β-sitosterol,
≤ 30 % by weight stigmasterol,
< 15 % by weight stigmastanol,
b) dissolves the phytosterol-phytostanol feed-stock composition of a) in a solvent,
c) hydrogenates the dissolved phytosterol-phytostanol feed-stock composition of b) using a nickel hydrogenation catalyst in a temperature range of 40 °C to 120 °C and at a pressure of 4,000 hPa to 50,000 hPa, and
d) isolates the formed phytosterol-phytostanol composition,
wherein the indications of weight refer to the total mass of phytosterols and phytostanols in their free, not derivatized form, and
wherein the phytostanols and phytosterols in the compositions are present in free, not derivatized form, as phytosterol derivatives and phytostanol derivatives substituted in 3-position, in their epimeric forms, or as the mixtures thereof.

2. Method according to claim 1, **characterized in that**, in step a) one provides a phytosterol-phytostanol feed-stock composition,
comprising
≥ 5 % by weight brassicasterol,
6 to 40 % by weight campesterol,
≤ 4 % by weight campestanol,
≤ 70 % by weight β-sitosterol,
≤ 10 % by weight stigmasterol and
≤ 10 % by weight stigmastanol,
wherein the indications of weight refer to the total mass of phytosterols and phytostanols in their free, not derivatized form, and
wherein the phytostanols and phytosterols in the composition are present in free, not derivatized form, as phytosterol derivatives and phytostanol derivatives substituted in 3-position, in their epimeric forms, or as the mixtures thereof.

3. Method according to any of the preceding claims, **characterized in that** in step a) one provides a phytosterol-phytostanol feed-stock composition, comprising
9 to 13 % by weight brassicasterol,
25 to 40 % by weight campesterol,
≤ 1 % by weight campestanol,
40 bis 60 % by weight β-sitosterol,
≤ 1 % by weight stigmasterol and
≤ 1 % by weight stigmastanol,
based on the total mass of phytosterols and phytostanols in their free, not derivatized form, wherein the phytosterols and phytostanols are present in free, not derivatized form, as phytosterol derivatives and phytostanol derivatives substituted in 3-position, in their epimeric forms, or as the mixtures thereof.

4. Method according to any of the preceding claims, **characterized in that** the phytosterol derivatives or phytostanol derivatives substituted in 3-position, are phytosterol esters, phytostanol esters, phytosterol ethers, phytostanol ethers or the mixtures thereof.

5. Method according to any of the preceding claims, **characterized in that** the phytosterol derivatives and phytostanol derivatives are esters of short-chain aliphatic carboxylic acids, or esters of vegetable oil-based fatty acids, or the mixtures thereof.

6. Method according to any of the preceding claims, **characterized in that** the phytosterol derivatives and phytostanol derivatives are phytosterol glycosides, phytostanol glycosides or the mixtures thereof.

7. Method according to any of the preceding claims, **characterized in that** in a further step e) one substitutes free, not derivatized phytosterols and phytostanols present in the phytosterol-phytostanol composition in the 3-position and converts them to their esters or ethers.

8. Method according to any of the preceding claims, **characterized in that** in a further step f) one blends the phytosterol-phytostanol composition obtained in step d) or e) with a phytosterol-phytostanol feed-stock composition according to the one used in step a).

9. Method according to claim 8, **characterized in that** in a further step g) one substitutes free, not derivatized phytosterols and phytostanols obtained in step f) in 3-position and converts them into their esters or ethers.

10. Method according to any of the preceding claims, **characterized in that** in step a) one provides as phytosterol-phytostanol feed-stock composition a phytosterol-phytostanol concentrate, obtained from deodorizer distillates of plant oil refining, distillation residues from the production of biodiesel, the esters, ethers or the mixtures thereof.

11. Method according to any of the preceding claims, **characterized in that** in step b) one dissolves the phytosterol-phytostanol feed-stock composition in an organic solvent, selected from alcohols and carbonic esters, and the mixtures thereof.

12. Method according to any of the preceding claims, **characterized in that** in step c) one performs the hydrogenation by use of an aluminous, activated nickel catalyst, and **in that** the nickel catalyst is promoted with iron, chromium or molybdenum, or is not promoted.

13. Method according to any of the preceding claims, **characterized in that** in step c) one utilizes the catalyst in an amount of 0.5 % by weight to 15 % by weight, based on the total mass of phytosterols and phytostanols in their free, not derivatized form, and/or in step c) one performs the hydrogenation in a temperature range of 60 °C to 100 °C and at a pressure of 25,000 to 35,000 hPa.

## Revendications

1. Procédé de fabrication d'une composition de phytostérols-phytostanols, comprenant
≤ 3 % en poids de brassicastérol,
≤ 40 % en poids de campestérol,
≤ 5 % en poids de campestanol,
≤ 80 % en poids de β-sitostérol,
≤ 30 % en poids de stigmastérol,
≤ 15 % en poids de stigmastanol, et
≤ 3 % en poids d'autres stérols,
dans lequel
a) on met à disposition une composition de phytostérols-phytostanols de départ, comprenant
> 3 % en poids de brassicastérol,
3 à 40 % poids de campestérol,
< 5 % en poids de campestanol,
≤ 80 % en poids de β-sitostérol,
≤ 30 % en poids de stigmastérol,
< 15 % en poids de stigmastanol,
b) on dissout la composition de phytostérols-phytostanols de départ obtenue en a) dans un solvant,
c) on soumet la composition de phytostérols-phytostanols de départ dissoute obtenue en b) à une hydrogénation réalisée en mettant en œuvre un catalyseur d'hydrogénation au nickel dans une gamme de températures comprise entre 40 °C et 120 °C et sous une pression comprise entre 4 000 hPa et 50 000 hPa, et
d) on isole la composition de phytostérols-phytostanols ainsi formée,
les indications de poids se référant à la masse totale de phytostérols et phytostanols sous leur forme libre non-dérivatisée, et
les phytostérols et phytostanols étant présents, au sein desdites compositions, sous une forme libre non-dérivatisée, sous forme de dérivés de phytostérol et dérivés de phytostanols substitués en position 3, sous leurs formes épimères ou sous forme de mélanges desdits éléments.

2. Procédé selon la revendication 1, **caractérisé en ce qu'**à l'étape a), on met à disposition une composition de phytostérols-phytostanols de départ,
comprenant
≥ 5 % en poids de brassicastérol,
6 à 40 % poids de campestérol,
≤ 4 % en poids de campestanol,
≤ 70 % en poids de β-sitostérol,
≤ 10 % en poids de stigmastérol, et
≤ 10 % en poids de stigmastanol,
les indications de poids se référant à la masse totale de phytostérols et phytostanols sous leur forme libre non-dérivatisée, et
les phytostérols et phytostanols étant présents, au sein de ladite composition, sous une forme libre non-dérivatisée, sous forme de dérivés de phytostérol et dérivés de phytostanols substitués en position 3, sous leurs formes épimères ou sous forme de mélanges desdits éléments.

3. Procédé selon l'une des revendications précédentes, **caractérisé en ce qu'**à l'étape a), on met à disposition une composition de phytostérols-phytostanols de départ, comprenant
9 à 13 % en poids de brassicastérol,
25 à 40 % poids de campestérol,
≤ 1 % en poids de campestanol,
40 à 60 % en poids de β-sitostérol,
≤ 1 % en poids de stigmastérol, et
≤ 1 % en poids de stigmastanol,
par rapport à la masse totale de phytostérols et phytostanols sous leur forme libre non-dérivatisée, les phytostérols et phytostanols étant présents sous une forme libre non-dérivatisée, sous forme de dérivés de phytostérol et dérivés de phytostanols substitués en position 3, sous leurs formes épimères ou sous forme de mélanges desdits éléments.

4. Procédé selon l'une des revendications précédentes, **caractérisé en ce que** les dérivés de phytostérol et dérivés de phytostanols substitués en position 3 correspondent à des esters de phytostérol, esters de phytostanol, éthers de phytostérol, éthers de phytostanol ou à des mélanges desdits éléments.

5. Procédé selon l'une des revendications précédentes, **caractérisé en ce que** les dérivés de phytostérol et dérivés de phytostanols correspondent à des esters d'acides carboxyliques aliphatiques à courte chaîne ou des esters d'acides gras issus d'huiles végétale, ou à des mélanges desdits éléments.

6. Procédé selon l'une des revendications précédentes, **caractérisé en ce que** lesdits dérivés de phytostérol et dérivés de phytostanols sont des glycosides de phytostérols, des glycosides de phytostanols, ou à des mélanges desdits éléments.

7. Procédé selon l'une des revendications précédentes, **caractérisé en ce que**, dans une étape supplémentaire e), on soumet les phytostérols et phytostanols libres non-dérivatisés, tels qu'ils sont contenus dans ladite composition de phytostérols-phytostanols, à une substitution en position 3 et on les transforme en leurs esters ou éthers.

8. Procédé selon l'une des revendications précédentes, **caractérisé en ce que**, dans une étape supplémentaire f), on mélange la composition de phytostérols-phytostanols telle qu'obtenue à l'étape d) ou e) avec une composition de phytostérols-phytostanols de départ conforme à la composition de phytostérols-phytostanols de départ mise en œuvre à l'étape a).

9. Procédé selon la revendication 8, **caractérisé en ce que**, dans une étape g), on soumet les phytostérols et phytostanols libres non-dérivatisés, tels qu'obtenus à l'étape f), à une substitution en position 3, et on les transforme en leurs esters et éthers.

10. Procédé selon l'une des revendications précédentes, **caractérisé en ce qu'**à l'étape a), on met à disposition en tant que composition de phytostérols-phytostanols de départ, un concentré de phytostérols-phytostanols obtenu à partir de distillats issus du traitement à la vapeur dans le cadre du raffinage des huiles végétales, de résidus de distillation issus de la fabrication du biodiesel, de leurs esters, de leurs éthers ou de mélanges desdits éléments.

11. Procédé selon l'une des revendications précédentes, **caractérisé en ce qu'**à l'étape b), on dissout la composition de phytostérols-phytostanols de départ dans un solvant organique choisi parmi les alcools ou les esters d'acides carboxyliques et leurs mélanges.

12. Procédé selon l'une des revendications précédentes, **caractérisé en ce qu'**à l'étape c), on réalise ladite hydrogénation en mettant en œuvre un catalyseur au nickel activé contenant de l'aluminium, ledit catalyseur au nickel pouvant comporter des promoteurs de type fer, chrome ou molybdène ou pouvant en être dépourvu.

13. Procédé selon l'une des revendications précédentes, **caractérisé en ce qu'**à l'étape c), on met en œuvre ledit catalyseur dans une quantité comprise entre 0,5 % en poids et 15 % en poids, par rapport à la masse totale de phytostérols et phytostanols se présentant sous leur forme libre non-dérivatisée, et/ou à l'étape c), on réalise ladite hydrogénation dans une gamme de températures comprise entre 60 °C et 100 °C et à une pression comprise entre 25 000 et 35 000 hPa.
